# EUROPEAN PATENT APPLICATION

(11) **EP 1 970 117 A1**
(43) Date of publication of application: **17.09.2008**
(21) Application number: 07103478.9
(22) Date of filing: 05.03.2007
(51) Int. Cl.: B01J 21/00, B01J 21/04, B01J 23/00, B01J 23/52, B01J 23/755, B01J 37/02, B01J 37/03, C07C 5/05, C07C 5/08, C07C 7/167, C10G 45/34

(54) **Gold-based catalysts for selective hydrogenation of unsaturated compounds**

(71) Applicant: Institut Catala D'Investigacio Quimica, 43007 Tarragona (ES)
(72) Inventor: Pérez-Ramirez, Javier, 43007, Tarragona (ES); Segura, Yolanda, 43007, Tarragona (ES)
(74) Representative: Barlocci, Anna

(57) **Abstract**

The present invention relates to novel selective hydrogenation gold-based catalysts for carrying out selective hydrogenation of molecules having triple bonds, and optionally also conjugated double bonds and/or triple and double bonds in the same molecule. The catalysts show a high selectivity and activity, and are also stable over time.

## Description

The present invention relates to a selective hydrogenation catalyst, more particularly to a supported gold catalyst for the selective hydrogenation of molecules having triple bonds, molecules having conjugated double bonds, and/or molecules bearing both triple and double bonds. Examples of such molecules include alkynes and conjugated alkadienes of an olefinic feed stream. Therefore, the catalyst of the invention has importance not only in the petrochemical industry, but also in fine and specialty chemicals. The present invention, also encompasses a process for the preparation of the catalyst herein described.

### BACKGROUND ART

The selective hydrogenation of alkynes and conjugated alkadienes to the corresponding mono-olefin is an industrially relevant process for hydrorefining of C₂, C₃, C₄, and gasoline cuts produced by steam cracking. The C₂ cut typically contains 90% of ethylene and 0.5-3% of acetylene, while the C₃ cut contains 90% of propylene and 2-8% of propyne and propadiene. These highly unsaturated compounds are undesirable in both chemical and polymer-grade propylene and ethylene, respectively.

Acetylenic impurities need to be removed from the streams to produce acceptable quality olefin and diene products. A preferred technique for removing the acetylenic impurities is the selective hydrogenation. The difficulty in the catalytic hydrogenation of acetylenic compounds and dienes arises from the fact that the hydrogenation must be carried out in the presence of large amounts of olefins. The reason is that under industrial conditions, valuable olefin products in the crude product streams are not inert.

Palladium catalysts are the choice of all current commercial processes for the selective hydrogenation of acetylenic impurities in crude butadiene and crude C₃ olefin streams.

Others metallic catalysts have been reported to be useful for the hydrogenation of acetylenes such us platinum, nickel or cobalt. However, beside palladium, no other metal catalyst is industrially used due to their insufficient selectivity. Pd catalysts are industrially the most preferred because of high activity and supposedly superior selectivity compared with other metal catalysts.

Unfortunately, the hydrogenation process over Pd-based catalysts is far to be optimal regarding to selectivity, due to the over-hydrogenation reaction to the corresponding alkane. Besides, oligomerization leads to lower alkene selectivities and green oil formation which diminishes the catalyst lifetime.

Few documents have been reported with the combination of different metals, where the active phase (typically Pd) is promoted by the addition of small amounts of Au, Ag, Ge, Sn, Pb, Ga, In, etc. However, although the selectivity is increased with the presence of such promoters, the activity is reduced.

One of the most serious problems in the hydrogenation reaction of compounds having a triple bond and/or conjugated double bonds, is catalyst deactivation. Said deactivation occurs by deposition of oligomers generated in the reactor (called green oil) on the catalyst; the H₂/C₂H₂ ratio is the main parameter that affects oligomerization; and the elevation in the reaction temperature also contributes to the oligomer formation.

To compensate the catalyst deactivation, it is necessary to gradually increase the reactor inlet temperature. However, the gradual increase in temperature decreases selectivity for ethylene in favor of formation of ethane, which is a product with a commercial value about five times lower than that of ethylene. During the reactor runs, the ethylene selectivity decreases until reaching a limiting value when the catalyst bed is replaced or regenerated.

Gold-based catalysts have also been reported for alkyne and alkadiene hydrogenation. For example, high ethylene selectivities have been achieved (>90%) at 40-250 °C during the ethyne hydrogenation over Au/Al₂O₃ catalysts (Jia et al. J. Phys. Chem. B 2000, 104, 11153-11156), wherein ultrafine gold particles were prepared on alumina support by a deposition-precipitation method; Goodman et al (Catal. Lett. 2003, 86, 1-8) have investigated on Au/TiO₂ and Au-Pd/TiO₂ catalysts the acetylene hydrogenation obtaining >50% selectivities at 70 °C; Haruta et al. (Catal. Today 2002, 74, 265-269) have also reported the hydrogenation of 1,3- butadiene over dispersed gold supported on Al₂O₃, SiO₂ and TiO₂ supports, obtaining selectivities of 75% at 106-277 °C; Lennon et al. (Appl. Catal. A: General 2005, 291, 230-237) investigated the hydrogenation of propyne over Au/TiO₂ and Au/Fe₂O₃ at 250-350 °C by using a pulse method. The reaction led to propylene as the only product at the reactor outlet, with propyne conversions up to 40%. US4299800 patent discloses the use of a gold catalyst for the selective reduction/hydrogenation of oxygen in an olefin stream; and US5506273 relates to the use of gold for the hydrogenation of CO and CO₂ to produce methanol and hydrocarbons.

The International application WO03106021 discloses a gold impregnated catalyst on a high surface area support for selectively hydrogenation of acetylene in an olefinic feed stream, particularly for ethylene purification. The emphasis of this patent application is the low concentration of gold (<0.5 %) and the importance of a high surface area (>150 m²/g) of an inert carrier. The carrier may be any high surface area catalyst support, such as alumina, silica-alumina, zinc oxide, nickel spinel, titania, zirconia, ceria, chromia-alumina, magnesium oxide, cerium oxide and mixtures thereof. The preferred carrier is high surface area alumina. The catalysts can also include small quantities of a noble metal, preferably palladium to improve the performance. Best selectivities to ethylene are obtained for a Au/Al₂O₃ catalyst: 41,9 % (activity range: 93,8 °C) and particularly when palladium is added as an additive, thus for a Au/Pd/Al₂O₃ catalysts the best selectivity obtained is 57,7% (activity range: 55-67,8 °C)

According to Jia et al. (J. Phys. Chem. B 2000, 104, 11153-11156), ultrafine gold particles of Au/Al₂O₃ catalyst are regarded as a unique metal for the selective hydrogenation only to ethylene from acetylene. The weak adsorption of ethylene on the surface of the gold nanoparticles of Au/Al₂O₃ catalyst may be the reason for its poor hydrogenation activity, the conversion rates obtained of ethyne to ethylene is over 2000 times higher than that of hydrogenation of ethylene. Furthermore, the process described in this paper corresponds to a batch process, which is not suitable for industrial application.

A disadvantage of using gold as catalyst is that gold particles tend to agglomerate, and when the particle size of around 10 nm, its capability to adsorb acetylene and H₂ is practically null, and then the catalyst is inactive.

Therefore, due to the tendency of gold to agglomerate, its activity decreases over time.

Accordingly, it is therefore desirable to provide a catalyst for the selective hydrogenation of a triple bond, a conjugated double bond and/or a triple bond in presence of a double bond in the same compound, to overcome the known disadvantages of the known catalysts.

### SUMMARY OF THE INVENTION

The inventors have found that using a gold-based catalyst where the support contains at least one divalent transition metal and at least other metal element which is a trivalent metal, high selectivity, activity and stability is achieved in the hydrogenation of alkynes and conjugated alkadienes in an olefinic feed stream.

Particularly, inventors have found a new gold-based catalyst for selective hydrogenation of a triple bond, a conjugated double bond and/or a triple bond in presence of a double bond in the same compound, which shows a high selectivity and activity, and which also is stable over time, i.e. it does not suffers from deactivation.

Thus, an aspect of the present invention is the provision of a gold-based catalyst which comprises gold particles co-precipitated jointly or deposited on a non-inert support selected from a mixed multimetallic oxide; a co-precipitated of at least two different metal oxides; and mixtures thereof; where at least one metal element is a divalent transition metal and where at least other metal element is a trivalent metal.

Compared with other immobilized gold-based catalysts known in the art, excellent results have been obtained regarding to operating temperature, selectivity and stability over time. Thus, the catalyst of the invention is a stable compound, which maintains its activity during a long time and with high selectivity, working at a suitable operation temperature.

According to another aspect, the invention provides a process for the preparation of the catalyst of the invention which comprises the steps: a1) providing a non-inert support, where said support comprises a mixed multimetallic oxide; a co-precipitated of at least two metal oxides; or mixtures thereof; where at least one metal element is a divalent transition metal and where at least other metal element is a trivalent metal; b1) depositing and precipitating the gold particles on the support by adding the support of step (a1) to an aqueous solution of an inorganic salt of gold and urea; and c1) calcinating the catalyst thus obtained.

Alternatively, the process comprises the steps: a2) co-precipitating an aqueous solution of an inorganic salt of with an aqueous solution of at least two inorganic salts of different metals; where at least one metal is a divalent transition metal and where at least other metal is a trivalent metal, with a precipitating agent; and b2) calcinating the catalyst obtained in step a2).

According to another aspect of the invention, it is provided a process for the selective hydrogenation of a triple bond, a conjugated double bond and/or a triple bond in presence of a double bond in the same compound, which comprises contacting the gold-based catalyst of the invention with a gaseous feed stream of the compound or a solution in a suitable organic solvent of the compound, and a hydrogen stream.

According to another aspect of the invention, it is provided the use of the product as defined above as a catalyst for the selective hydrogenation of a triple bond, a conjugated double bond and/or a triple bond in presence of a double bond in the same compound.

### DETAILED DESCRIPTION OF THE INVENTION:

According to the present invention, high selectivity, conversion rates and stability of the catalyst results from the suitable combination of a proper non-inert support and gold. It is crucial the cooperation between gold nanoparticles and the active support catalyst.

According to an embodiment of the invention, the catalysts of the invention are useful for selective hydrogenation of alkynes and conjugated alkadienes in an olefinic feed stream. Preferably, the catalyst of the invention is useful for selective hydrogenation of (C₂-C₅)-alkynes and conjugated (C₂-C₅)-alkadienes in an olefinic feed stream. When the compound is a (C₂-C₅)-alkyne and/or conjugated (C₂-C₅)-alkadiene, the process preferably comprises passing a conventional (C₂-C₅)-alkyne and/or conjugated (C₂-C₅)-alkadiene feed stream in combination with a H₂ stream over the catalyst of the invention, and recovering the resulting (C₂-C₅)-alkenes.

According to an embodiment of the present invention, the gold-based catalyst comprises at least one metal element which is a divalent transition metal selected from nickel, manganese, cobalt, cadmium, magnesium, copper and zinc.

According to an embodiment of the present invention, the gold-based catalyst comprises at least other metal element which is a trivalent metal selected from aluminum, gallium, chromium and iron.

Therefore, the support contains at least one divalent transition metal and at least one trivalent metal. Preferably, the mixed multimetallic oxide of the support comprises between 2 and 5 different metals, more preferably between 2 and 3 different metals. Also, preferably, the combination of at least two metal oxides of the support comprises between 2 and 5 different metal oxides, more preferably 2 and 3 different metal oxides. Also preferably, the support comprises mixtures of the above preferred mixed multimetal oxides and combination of different metal oxides.

In a particular embodiment, the support may contain only one divalent transition metal and only one trivalent metal.

According to a preferred embodiment of the invention, at least one of the divalent transition metal is nickel. According to an embodiment of the invention, when the support comprises more than one divalent transition metal and/or more than one trivalent metal, nickel is present in the composition of the support.

According to a preferred embodiment of the invention, at least one of the trivalent metal is aluminum.

In another preferred embodiment, the gold-based catalyst of the invention contains as non-inert support a mixed NiAl oxide, a combination of nickel and aluminium oxides, or mixtures thereof.

The preferred support has a surface area greater than 150 m²/g, preferably from 125 m²/g to 1000 m²/g, more preferably from 150 m²/g to 250 m²/g.

Preferably the concentration of the gold in the catalyst is from 0.05 to 5 weight percent based on the total weight of the catalyst, preferably between 1 to 3 weight percent, more preferably 2 weight percent.

The size of the gold particles depends on the gold calcination temperature, becoming larger as the temperature is increased; particle size is measured by transmission electron microscopy (TEM).

Generally, the particle size of the gold particles present in the catalyst of the invention is below 10 nm. Preferably, the particle size of the gold particles is between 2-8 nm, particularly preferably between 2-5 nm.

When the catalytic hydrogenation is carried out at temperatures between 200 °C and 300 °C, the preferred particle size of the gold particles present in the catalyst is between 5-8 nm. However, in order to maintain the high selectivity and conversion rates of the catalytic hydrogenation, when it is carried out at temperatures between 100 °C and 200 °C, the particle size of the gold particles is preferably in the range of 3-6 nm.

The catalyst can be obtained by a process which comprises the steps: a1) providing a non-inert support, wherein said support comprises a mixed multimetallic oxide; a co-precipitated of at least two metal oxides; or mixtures thereof; where at least one metal element is a divalent transition metal and where at least other metal element is a trivalent metal; b1) depositing and precipitating the gold particles on the support by adding the support of step (a1) to an aqueous solution of an inorganic salt of gold, such as HAuCl₄, and urea; and c1) calcinating the catalyst thus obtained. The calcination generally ranges from 150-550 °C. Preferably from 200-450 °C, particularly preferred from 225 to 375 °C.

Alternatively, the catalyst can be obtained by a process which comprises the steps: a2) co-precipitating an aqueous solution of an inorganic salt of with an aqueous solution of at least two inorganic salts of different metals, such as Ni(NO₃)₂ 6H₂O, Al(NO₃)₃ 9H₂O; where at least one metal is a divalent transition metal and where at least other metal is a trivalent metal, with a precipitating agent; and b2) calcinating the catalyst obtained in step a2). An example of an appropriate precipitating agent is (NaOH+Na₂CO₃). The calcination generally ranges from 150 to 550 °C. Preferably from 200-450 °C, particularly preferred from 225 to 375 °C.

In an embodiment of the invention, the non-inert support provided in step a1) is undergone to a calcination process before its addition to the aqueous solution of gold in step a2). Preferably, the calcination temperature of the support ranges from 300-550 °C. Alternatively, gold can be deposited on the non calcined support, and then undergone the catalyst to the calcination step c1).

Optionally, once the catalyst is prepared (after steps c1) or b2), an additional pre-treatment step can be applied. Such pre-treatment step may consist in a reduction of the catalyst by a flow of hydrogen in an inert gas (e.g. helium). Such pre-treatment step is carried out at a temperature between 150 and 500 °C, preferably between 200 and 300 °C.

The catalytic reaction can be carried out in a batch process (in a slurry reactor) or in a continuous process (trickle flow). Depending of the nature of the compound, the catalytic reaction can be carried out using a gaseous feed stream of the compound or using a liquid solution of the compound in a suitable organic solvent.

As mentioned above, the catalyst of the invention is useful for the hydrogenation of (C₂-C₅)-alkynes and conjugated (C₂-C₅)-alkadienes in an olefinic feed stream. In an embodiment of the invention, the hydrogenation of (C₂-C₅)-alkyne or conjugate (C₂-C₅)-alkadiene reaction is carried out in presence of an excess of H₂, preferably with an (C₂-C₅)-alkyne or conjugate (C₂-C₅)-alkadiene to H₂ ratio ranging between 1:5 and 1:1, more preferably 1:3.

Any suitable reaction pressure can be used. Preferably, the total pressure applied is in the range between atmospheric pressure and 20 bar. In a particular embodiment the total pressure applied is the atmospheric pressure.

Preferably the temperature of hydrogenation is in the range of 100-350 °C, more preferably between 120 and 250 °C. The process of hydrogenation results in excellent ratios of selectivity and activity when the process is carried out at temperatures between 150 °C and 250 °C.

Generally, the gold catalyst is placed in a quartz micro-reactor. The catalyst sample is pretreated in a mixture of H₂ in He at temperature ranging from 200-500 °C. The catalyst is tested in the range 100-350 °C. The product gases are analyzed using gas chromatography.

As used herein, the following terms are used to describe the present invention. The definitions provided below, within context, may be used exclusively, or may be used to supplement definitions which are generally known to those of ordinary skill in the art.

### DEFINITIONS

Throughout the description and claims the word "comprise" and variations of the word, such as "comprising", are not intended to exclude other technical features, additives, components, or steps.

In the context of the invention, the term "selectivity" refers to the selectivity to alkene, and it was determined as the amount of alkene formed divided by the amount of alkyne reacted.

In the context of the invention, the term "activity" is interchangeable with the term "conversion rate", and denotes the amount of alkene formed from a determined amount of alkyne as started material.

The term "total pressure" refers to the total pressure applied throughout the reactor.

The term "gas hourly space velocity" (GHSV) refers to the total gas flow treated in an hour divided by the volume of catalyst.

The term "calcination" refers to a thermal treatment process applied to solid materials in order to bring about a thermal decomposition, phase transition, or removal of a volatile fraction.

Additional objects, advantages and features of the invention will become apparent to those skilled in the art upon examination of the description or may be learned by practice of the invention. The following examples and drawings are provided by way of illustration, and is not intended to be limiting of the present invention.

### BRIEF DESCRIPTION OF THE DRAWINGS

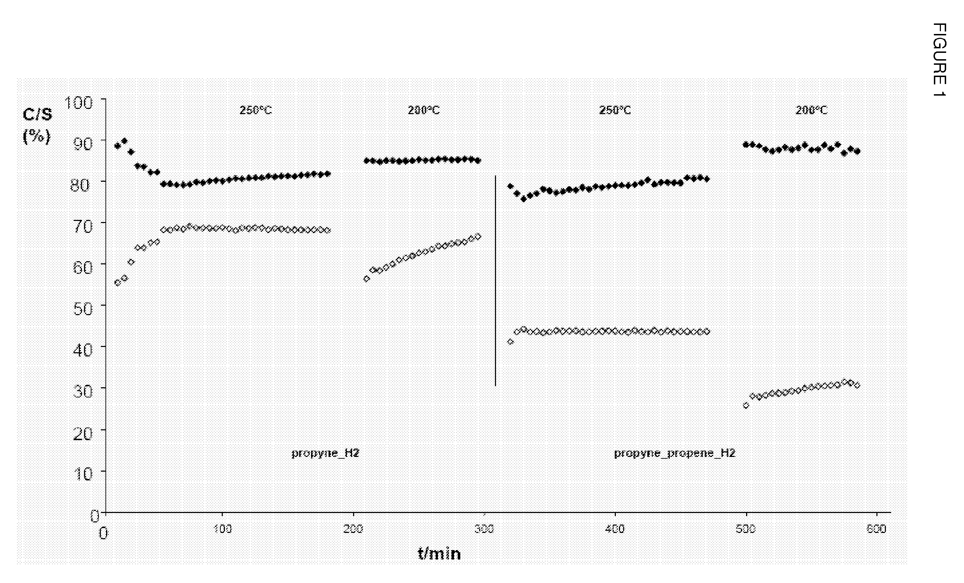

FIGURE 1 shows the selectivity to propene and the conversion of propyne hydrogenation at the reaction temperature of 250-200 °C, with and without the addition of propene to the propyne:hydrogen mixture.

| | |
|---|---|
| ◇ | Conversion of propyne |
| ◆ | Selectivity to propene |
| C / S | Conversion or Selectivity % |
| t / min | Time on stream / min |

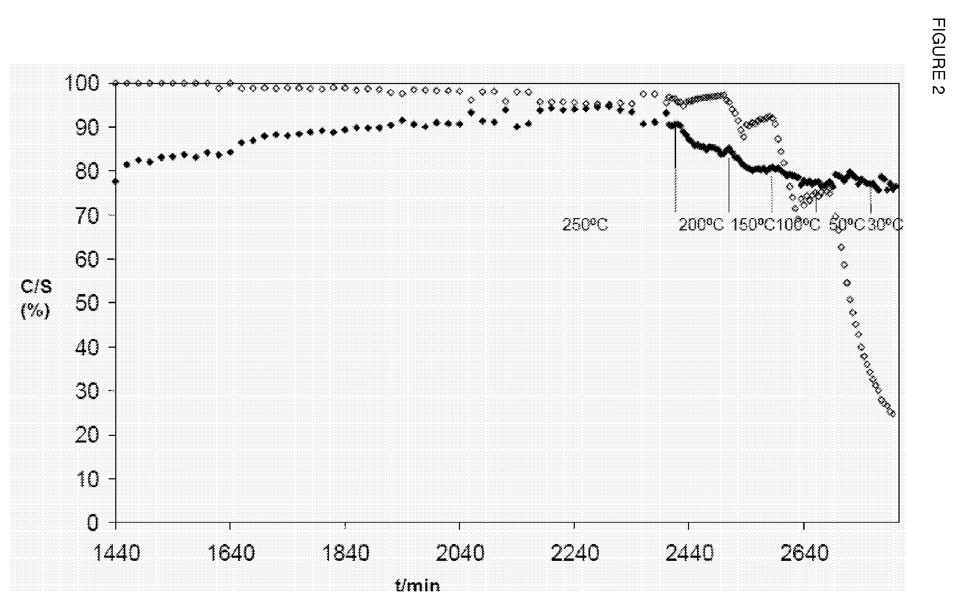

FIGURE 2 shows that Au/NiAl oxide catalyst is stable over time on stream. At 250 °C (after 24 hours on stream) selectivity and conversion remain constant.

| | |
|---|---|
| ◇ | Conversion to propyne |
| ◆ | Selectivity to propene |
| C / S | Conversion or Selectivity % |
| t / min | Time on stream / min |

### DETAILED DESCRIPTION OF PARTICULAR EMBODIMENTS

### Example 1 Preparation of a non-inert support of NiAl mixed oxide.

A NiAl mixed oxide support was prepared with nominal metal molar ratios of M²⁺/M³⁺ of 3:1. It was prepared by co-precipitation at constant pH using the in-line dispersion-precipitation (ILDP) method. Briefly, aqueous solutions of the metal nitrate Ni(NO₃)₂·6H₂O and Al(NO₃)₃·9H₂O, and the precipitating agent (NaOH+Na₂CO₃) were continuously fed at room temperature by means of peristaltic pumps into a home-made micro-reactor with an effective volume of ca. 6 cm³. An in-line probe measured the pH of the slurry directly at the outlet of the micro-reactor and was connected to one of the pumps to keep constant pH = 10. The micro-reactor was stirred at 13,500 rpm by means of a highspeed disperser and the residence time was fixed at 36 s. The resulting slurry was aged in a glass vessel during 12 h at 25 °C under mechanical stirring (500 rpm). Finally, the material was filtered, thoroughly washed with deionized water to remove Na⁺ and NO³⁻ ions, and dried at 80 °C for 12 h obtaining a surface area catalyst support of (at least) 180 m²/g (measure by N₂-sorption analysis).

### Example 2 Preparation of the gold-based catalyst by the co-precipitation method.

A Au-based catalyst was synthesized by the co-precipitation method.
The Au/NiAl hydrotalcite with nominal metal molar ratios of M²⁺/M³⁺ of 3:1 and a nominal gold metal content of 2 wt.% was prepared by co-precipitation at constant pH using the in-line dispersion-precipitation (ILDP) method. Briefly, aqueous solutions of the respective metal nitrates Ni(NO₃)₂·6H₂O, Al(NO₃)₃·9H₂O, and HAuCl₄·3H₂O, and the precipitating agent (NaOH+Na₂CO₃) were continuously fed at room temperature by means of peristaltic pumps into a home-made micro-reactor with an effective volume of ca. 6 cm³. An in-line probe measured the pH of the slurry directly at the outlet of the micro-reactor and was connected to one of the pumps to keep constant pH = 10. The micro-reactor was stirred at 13,500 rpm by means of a highspeed disperser and the residence time was fixed at 36 s. The resulting slurry was aged at 25 °C under stirring (500 rpm) in a glass vessel during 12 h. Finally, the material was filtered, thoroughly washed with deionized water to remove Na⁺ and NO³⁻ ions, and dried at 80 °C for 12 h. The as-synthesized sample was calcined in static air at 450 °C for 15 h obtaining a surface area of (at least) 175 m²/g (measure by N₂-sorption analysis). The gold particle sizes are in the range of 3-8 nm (by TEM analysis)

### Example 3 Preparation of the gold-based catalyst by the deposition-precipitation method.

A catalyst was prepared by the deposition-precipitation method using urea as the precipitating agent. For that purpose, 1 g of a previously synthesized NiAl hydrotalcite (prepared according to procedure of Example 1) was added to 45 ml of an aqueous solution of HAuCl₄ (1.4 10-3) and of urea (0.42 M). The suspension was heated at 80 °C and vigorously stirred for 4 h, filtered, washed with water, dried and calcinated at 450 °C. The surface area of the catalyst obtained is (at least) 175 m²/g (measure by N₂-sorption analysis). The gold particle sizes are in the range of 3-8 nm (by TEM analysis)

### Example 4 (Comparative example). Preparation of a gold-based catalyst where the support is prepared by a conventional dry-impregnation method.

A catalyst was prepared by the incorporation of gold by the deposition-precipitation method using urea as the precipitating agent (according to the procedure of Example 3). Gold is deposited on a mixed oxide support obtained by the conventional dry-impregnation method of NiOₓ on Alumina. A solution of nickel nitrate is impregnated on a dried solid alumina. After the impregnation, the catalyst was dried at 60 °C during 6 h and then calcined at 500 °C for 4 h. The surface area of the catalyst obtained is (at least) 107 m²/g (measure by N₂-sorption analysis). The gold particle sizes are in the range of 3-8 nm (by TEM analysis)

### Example 5. (Comparative example). Preparation of a gold-based catalyst where the gold is incorporated by dry impregnation.

A catalyst was prepared by the incorporation of gold by dry impregnation on a NiAl mixed oxide (prepared according to procedure of Example 1). The surface area of the catalyst obtained is (at least) 146 m²/g (measure by N₂-sorption analysis). The gold particle sizes are in the range of 20-80 nm (by TEM analysis)

### Example 6. Hydrogenation tests.

The catalysts of examples 1-5 were tested at 250 °C of temperature and their selectivity and conversion rates were measured. Table 1 contains the results obtained.

In use, the gold catalyst was placed in a quartz micro-reactor (12 mm id). The catalyst sample (0.15 g, sieve fraction 125-300 µm) was pretreated in a mixture of 15 vol. % H₂ in He at 250 °C during 1 h. The catalyst was tested in the range 100-300 °C using feed mixtures of C₃H₄/H₂/He = 2.5/7.5/90 and C₃H₄/C₃H₆/H₂/He = 2.5/2.5/7.5/90, a total gas flow rate of 42 ml (STP) min⁻¹ and a gas hourly space velocity (GHSV) of 16800 h⁻¹. Experiments were carried out at atmospheric pressure. The product gases were analyzed using gas chromatography.

**Table 1. Selectivity and Conversion rates obtained with the catalyst of examples 1-5.**

| Example | Au content approx. (wt %) | Nominal content support M²⁺ : M³⁺ | Selectivity (%) | Conversion (%) |
|---|---|---|---|---|
| Ex.1 (*NiAl*) | 0 | 3 | 70 | 50 |
| *Ex.2 (Au*/*NiAl(cop))* | 2 | 3 | 95 | 95 |
| *Ex.3 (Au(u)*/*NiAl)* | 2 | 3 | 90 | 95 |
| *Ex.3 (Au(u)*/*NiAl (400 ºC)* | 2 | 3 | 60 | 90 |
| *Ex.4 (Au(u)*/*Ni(imp)Al)* | 2 | 0.25 | 13 | 15 |
| *Ex.5 (Au(imp)*/*NiAl)* | 2 | 3 | 35 | 10 |

| | | | | |
|---|---|---|---|---|
| *Au* / *NiAl (cop):* where gold and support have been synthesized by the Co-precipitation method. *Au(u)*/ *NiAl* : where gold has been introduced by the Urea method. *Au(u)*/ *NiAl (400 ºC*): where the catalyst has been reduced at 400 °C before the catalytic test. The rest of the samples have been reduced at 250 °C. *Au(u)* / *Ni (imp)Al:* gold has been deposited by the urea method on a support where Ni was Impregnated on alumina. *Au(imp)* /*NiAl:* gold has been introduced by the Impregnation method on a mixed oxide NiAl support. | | | | |

### Example 7. Effect of hydrogen: propyne ratio feed mixture on the catalytic activity over Au/NiAl catalyst

The effect of hydrogen: propyne ratio feed mixture on the catalytic activity (selectivity and conversion) over a Au/NiAl catalyst of the invention was tested and Table 2 contains the results obtained. Best results were obtaining when the ratio is 1:3.

**Table 2. Effect of hydrogen: propyne ratio feed mixture on the catalytic activity over Au/NiAl catalyst.**

| Ratio H₂:Propyne | Conversion of propyne (%) | Selectivity to propene (%) | Yield to propene (%) | Temperature (ºC) |
|---|---|---|---|---|
| 5 | 100 | 5 | 5 | 150 |
| 3 | 96 | 90 | 84 | 150 |
| 2 | 78 | 78 | 63 | 150 |
| 1 | 48 | 63 | 30 | 150 |

### Example 8. Effects of support and gold precursor calcination temperatures on the catalytic activity over a Au(u)/NiAl catalyst.

The effect of support and gold precursor calcinations temperatures on the catalytic activity over a Au/NiAl catalyst was studied, and the results obtained are included in Table 3.

**Table 3. Effects of support and gold precursor calcination temperatures on the catalytic activity over a Au(u)/NiAl catalyst.**

| Sample | T^{a} calcination support (°C) | T^{a} calcination catalyst (°C) | T^{a} pre-treatment (°C) | Selectivity to alkene (%) | Conversion of alkyne (%) |
|---|---|---|---|---|---|
| 1^{a} | 450 | 450 | 250 | 90 | 95 |
| 2^{a} | 450 | 450 | 400 | 60 | 90 |
| 3^{b} | 350 | 250 | 250 | 99 | 100 |
| 4^{b} | 350 | 350 | 250 | 92 | 100 |
| 5^{b} | 450 | 450 | 250 | 75 | 95 |

| | | | | | |
|---|---|---|---|---|---|
| ^{a} Feed H₂: C₃H₄ 3: 1, Temperature of the hydrogenation reaction: 250°C ^{b} Feed H₂: C₃H₄ 3: 1, Temperature of the hydrogenation reaction: 200°C T^{a} of pretreatment: Temperature of the reduction of the catalyst before the reaction | | | | | |

By comparing experiment 1 and 2, the effect of the pre-treatment temperature is studied. The best selectivity was obtained when catalyst is pretreated at 250 °C.

By comparing experiments 3, 4 and 5, the effect of gold sintering is studied. Best results were obtained when calcination temperature of the gold catalyst is kept low.

## Claims

1. A selective hydrogenation gold-based catalyst which comprises gold particles co-precipitated jointly or deposited on a non-inert support selected from the group consisting of: a mixed multimetallic oxide; a co-precipitated of at least two different metal oxides; and mixtures thereof; wherein at least one metal element is a divalent transition metal and wherein at least other metal element is a trivalent metal.

2. The gold-based catalyst according to claim 1, wherein at least one metal element is a divalent transition metal selected from the group consisting of nickel, cadmium, manganese, cobalt, magnesium, zinc and copper.

3. The gold-based catalyst according to any of the claims 1-2, wherein at least one divalent transition metal is nickel.

4. The gold-based catalyst according to any of claims 1-3, wherein at least one metal element is a trivalent metal selected from the group consisting of aluminum, gallium, chromium and iron.

5. The gold-based catalyst according to any of the claims 1-4, wherein at least one divalent transition metal is nickel and at least one trivalent metal is aluminum.

6. The gold-based catalyst according to any of the claims 1-5, wherein the non-inert support is selected from the group consisting of a mixed NiAl oxide, a co-precipitate of nickel and aluminum oxide, and mixtures thereof

7. The gold-based catalyst according to any of the claims 1-6, wherein the particle size of the gold particles present in the catalyst of the invention is below 10 nm.

8. The gold-based catalyst according to claim 7, wherein the particle size of the gold particles is comprised between 2-5 nm.

9. The gold-based catalyst according to any of the claims 1-8, wherein the support has a surface area from 125 m²/g to 1000 m²/g.

10. The gold-based catalyst according to claim 9, wherein the surface area ranges from 150 m²/g to 250 m²/g.

11. The gold-based catalyst according to any of the claims 1-10, wherein the concentration of gold in the catalyst is from 0.05 to 5 weight percent based on the total weight of the catalyst.

12. The gold-based catalyst according to claim 11, wherein the concentration of gold in the catalyst is from 1 to 3 weight percent based on the total weight of the catalyst

13. A process for the preparation of a catalyst as defined in any of the claims 1-12 which comprises:
a1) providing a non-inert support, wherein said support comprises a mixed multimetallic oxide; a co-precipitate of at least two metal oxides; or mixtures thereof; wherein at least one metal element is a divalent transition metal and wherein at least other metal element is a trivalent metal;
b1) depositing and precipitating the gold particles on the support by adding the support of step (a1) to an aqueous solution of an inorganic salt of gold and urea; and
c1) calcinating the catalyst thus obtained.
or alternatively:
a2) co-precipitating an aqueous solution of an inorganic salt of with an aqueous solution of at least two inorganic salts of different metals; wherein at least one metal is a divalent transition metal and wherein at least other metal is a trivalent metal, with a precipitating agent; and
b2) calcinating the catalyst obtained in step a2).

14. The process according to claim 13, wherein the calcination temperature ranges from 150-550 °C.

15. The process according to claim 14, wherein the calcination temperature ranges from 200-450 °C.

16. The process according to claim 15, wherein calcination temperature ranges from 225-375 °C.

17. The process according to any of the claims 13-16, further comprising a calcination process of the non-inert support of step a1) before its addition to the aqueous solution of gold in step b1).

18. The process according to claim 17, wherein the calcination temperature ranges from 300-550 °C.

19. The process according to any of claims 13-18, further comprising a pre-treatment step which comprises the reduction of the catalyst by a flow of hydrogen in an inert gas.

20. The process according to claim 19, wherein the pre-treatment step is carried out at a temperature between 150-500 °C.

21. A process for the selective hydrogenation of a triple bond, a conjugated double bond and/or a triple bond in presence of a double bond in the same compound, which comprises contacting the gold-based catalyst according to any of claims 1 to 12 with a gaseous feed stream of the compound or a solution of the compound in a suitable organic solvent, and a hydrogen stream.

22. A process for selective hydrogenation of (C₂-C₅)-alkynes and conjugated (C₂-C₅)-alkadienes in an olefinic feed stream, which comprises passing a conventional (C₂-C₅)-alkyne and/or conjugated (C₂-C₅)-alkadiene feed stream in combination with a H₂ stream over the catalyst as defined in claims 1-12, and recovering the resulting alkenes.

23. The process according to claim 22, wherein the hydrogenation reaction is carried out with an alkyne or conjugate alkadiene to H₂ ratio between 1:5 to 1:1.

24. The process according to claim 23 wherein the (C₂-C₅)-alkyne or conjugate (C₂-C₅)-alkadiene to H₂ ratio is 1:3.

25. The process according to any of the claims 21-24, wherein the process is carried out applying a pressure which is in the range between atmospheric pressure to 20 bar.

26. The process according to claim 25, wherein the total pressure applied is the atmospheric pressure.

27. Use of the catalyst according to any of the claims 1 to 12, as a catalyst for the selective hydrogenation of a triple bond, a conjugated double bond and/or a triple bond in presence of a double bond in the same compound.

28. Use of the catalyst according to any of the claims 1 to 12, as a catalyst for the selective hydrogenation of (C₂-C₅)-alkynes and conjugated (C₂-C₅)-alkadienes in an olefinic feed stream.
